# EUROPEAN PATENT SPECIFICATION

(11) **EP 3 488 368 B1**
(45) Date of publication and mention of the grant of the patent: **13.11.2024**
(21) Application number: 17745480.8
(22) Date of filing: 24.07.2017
(51) Int. Cl.: G16H 40/40, G16H 40/63, G16H 40/67, G16H 50/50

(54) **SYSTEMS AND METHODS FOR VALIDATING SENSOR DATA**
SYSTEME UND VERFAHREN ZUR VALIDIERUNG VON SENSORDATEN
SYSTÈMES ET PROCÉDÉS DE VALIDATION DE DONNÉES DE CAPTEURS

(30) Priority: 22.07.2016 GB 201612721
(43) Date of publication of application: 29.05.2019
(73) Proprietor: Ixico Technologies Limited, London EC1A 9PN (GB)
(72) Inventor: HILL, Derek Lionel Glendon, London EC1A 9PN (GB)
(74) Representative: Stratagem IPM Limited
(86) International application number: PCT/GB2017/052152
(87) International publication number: WO 2018/015770

(56) References cited:
- EP-A1- 2 394 571
- US-A1- 2012 092 286
- US-A1- 2015 106 020
- JOVANOV EMIL ET AL: "A wireless body area network of intelligent motion sensors for computer assisted physical rehabilitation", JOURNAL OF NEUROENGINEERING AND REHABILITATION, BIOMED CENTRAL, LONDON, GB, vol. 2, no. 1, 1 March 2005 (2005-03-01), pages 6, XP021010818, ISSN: 1743-0003, DOI: 10.1186/1743-0003-2-6
- ALLEN C R ET AL: "3-D motion system (data-gloves): application for Parkinson's disease", IEEE TRANSACTIONS ON INSTRUMENTATION AND MEASUREMENT, IEEE SERVICE CENTER, PISCATAWAY, NJ, US, vol. 52, no. 3, 1 June 2003 (2003-06-01), pages 662 - 674, XP011098607, ISSN: 0018-9456, DOI: 10.1109/TIM.2003.814702

## Description

### Field

The present invention relates to systems and methods for validating sensor data, particularly, but not exclusively, biosensor data.

### Background

In digital healthcare sensor applications there is a distinct difference between three types of data: i) consumer grade; ii) medical grade; and iii) research grade.

Consumer grade biosensor data, for example from smart phone apps, home-based sensors and wearable devices, can be collected in large volumes from widely adopted technologies. These devices have, however, often undergone very little validation to demonstrate how meaningful the measurements are, and to characterise their accuracy. Furthermore, it is not clear how stable the measurements are over time, as the software environment of the sensors is often continuously evolving. This can be because the software on the sensor itself (eg: on a wearable), or software in the ecosystems (smartphone app, smart phones OS, cloud servers etc.) is upgraded automatically. It is possible that one reason for this change in software environment is that the sensors or surrounding ecosystem may be "self learning" and alter their performance as they gather more data.

Medical grade biosensor data is collected as part of formal healthcare service provision. This data is normally collected by medical devices, with well characterised performance, and formally validated software that must be updated in a very controlled way. But this data is often limited and sparse compared with what can be obtained from consumer devices, and the devices have less functionality, are less user-friendly, and generally have shorter battery life.

Research grade biosensor data is collected in research studies e.g.: evaluating new drugs or devices or studying the natural history of diseases. The biosensors used in these studies may not be medical devices, but need to have well characterized performance, for example in order to power the study. Similarly the use of the device needs to be controlled to ensure comparable data from different subjects and over time. For all three types of biosensor, obtaining actionable information to inform decision making in clinical trials and patient management requires validation of the measurement for the specific intended purpose, and the ability to calibrate between different devices, e.g. following upgrades of software or hardware. Furthermore, it is desirable to replace one sensor with another one during the course of a clinical trial or the management of a patient, and to ensure the data from the original and replacement sensors is comparable.

The present invention seeks to solve the aforementioned problems by providing systems and methods for validating sensor data such as data obtained from devices including wearable devices and smart phones, for example.

US2012/092286 discloses a synthetic gesture trace generator, wherein a synthetic gesture trace is generated using a gesture synthesizer which may be implemented in software. The synthesizer receives a number of inputs, including parameters associated with a touch sensor to be used in the synthesis and a gesture defined in terms of gesture components. The synthesizer breaks each gesture component into a series of time-stamped contact co-ordinates at the frame rate of the sensor, with each time-stamped contact co-ordinate detailing the position of any touch events at a particular time. Sensor images are then generated from the time-stamped contact co-ordinates using a contact-to-sensor transformation function. Where there are multiple simultaneous contacts, there may be multiple sensor images generated having the same time-stamp and these are combined to form a single sensor image for each time-stamp. This sequence of sensor images is formatted to create the synthetic gesture trace.

### Summary

There is disclosed a system for validating biosensor data, the system comprising: i) means for collecting biosensor data from a subject; ii) means for reproducing subject behaviour and/or physiological activity of the subject and collecting data corresponding with such reproduced behaviour and/or physiological activity; and iii) means for detecting any deviation over time in biosensor data collected from the reproduced activity in (ii) in order to identify and/or correct sensor or software introduced deviations in biosensor data collected from the subject.

The ability to use data to support research and clinical practice, as this data is much richer and more available than traditionally available medical grade and research grade data, is highly advantageous in improving the quality of research, regulatory submissions and subject care. The system defined by this aspect of the invention enables professionals to use consumer grade data with confidence due to the ability to verify the accuracy of collected data regardless of the type or brand of biosensor used to collect data.

The system may further comprise means to apply a correction factor to any deviation identified between biosensor data collected from the subject and data corresponding to reproduced behaviour.

According to the present invention, there is provided a method of calibrating sensing data between different sensors of the same type, the method comprising: a) obtaining physiological data from a test subject through a first sensor; b) using said physiological data to program a device to replicate physiological attributes exhibited by the test subject; c) applying a second sensor to the device, the second sensor comprising following upgrades of software or hardware from the first sensor; d) using the device to replicate the test subject's physiological attributes; e) obtaining physiological data from the second sensor regularly over time; f) identifying any variation between the physiological data obtained by the first sensor and the physiological data obtained by the multiple measurements made over time with the second sensor; g) applying a correction factor to the physiological data obtained from the second sensor in the event that any variation between physiological data obtained from the first sensor and physiological data obtained from the second sensor exceeds a predetermined threshold; and h) using said correction factor to calibrate the second sensor.

There is also provided a method of simultaneously calibrating sensing data from multiple sensors of the same type, the method comprising: a) obtaining physiological data from a test subject through a first sensor; b) using said physiological data to program a device to replicate physiological attributes exhibited by the test subject; c) applying multiple additional sensors to the device, the multiple additional sensors comprising following upgrades of software or hardware from the first sensor; d) using the device to replicate the test subject's physiological attributes; e) obtaining physiological data from the additional sensors; f) identifying any variation between the physiological data obtained by the first sensor and the physiological data obtained by the additional sensors; g) applying a correction factor to the physiological data obtained from the additional sensors in the event that any variation between physiological data obtained from the first sensor and physiological data obtained from the additional sensors exceeds a predetermined threshold; and h) using said correction factor to calibrate the additional sensors.

Use of the terms patient and subject are used interchangeably throughout and may relate to clinical patients, athletes or placebo subject, for example.

### Figures

Embodiments of the inventions will now be described by way of reference to the following figures:
Figure 1 illustrates problems associated with uncontrolled data collected from consumer sensors;
Figure 2 illustrates a first method of validating sensor data;
Figure 3 illustrates a second method of validating sensor data.

### Description

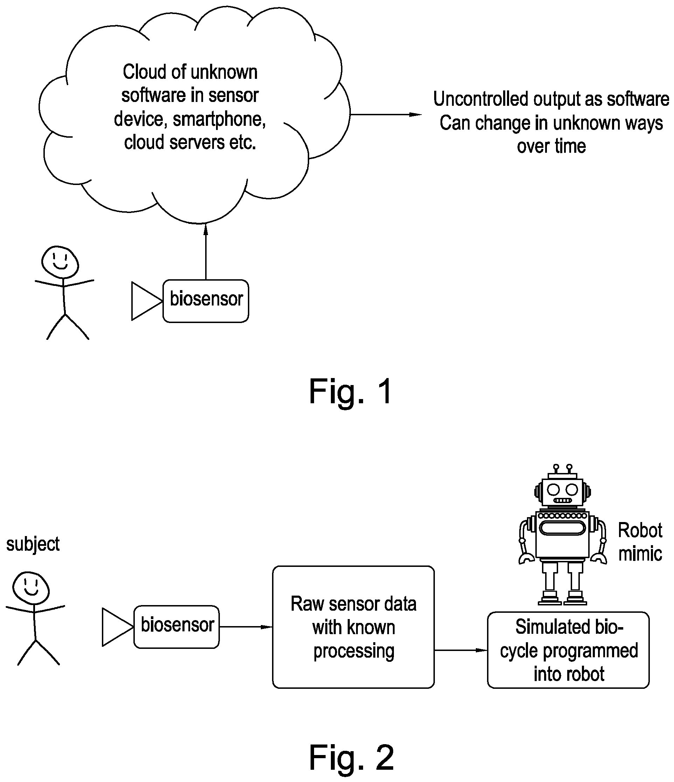

The technical problem is indicated schematically in figure 1. The biosensor (which might be a wearable, wall mounted or static device, or built into a smart-phone) makes measurements that then pass through a number of stages of processing on the sensor device, on any smart phone involved in the chain, and in any file server the data is transferred to. In figure 1, those various software processes and data transfers are represented as a "software cloud". The output is uncontrolled because what happens in the cloud can change in an unknown way at unknown times.

Key technical challenges need to be overcome to achieve the goal of using biosensors for regulated applications in clinical research and subject management, for example in clinical trials of new drugs, and as digital healthcare companion products to drugs. This requires addressing the issues of system validation, and quality assurance of data that is obtained out of this software cloud, in particular so any change over time in the outputs that is caused by the hardware software changes, device failure or improper use can be detected and corrected for.

Embodiments of the present invention describe a solution to the aforementioned technical challenges.

Rather than the validation of the biosensor being done before the device is used, with a carefully controlled process for upgrading software if any changes are required, the validation becomes a continuous process while data is being collected. This requires a standardised input of sensor data from a laboratory environment into the same software cloud that is handling the subject data.

This standardised input involves the use of a robot mimic. For the purposes of this application, "Robot Mimic" means a computer controlled system that, when connected to or measured by the sensor, generates sensor data that is highly correlated with that generated by the clinical trial subject/patient when connected to or measured by the same sensor. Figure 2 illustrates one example of how the robot mimic may be set up using actual biosensor data. Raw data from the biosensor is used to set up a simulated bio-cycle in the robot mimic.

The simulated bio-cycle determined using the approach illustrated in figure 2 can be run through the robot mimic as often as required and the robot mimic has sufficient reproducibility to ensure that it performs with a reproducibly that is much better than the measurements accuracy required by the application.

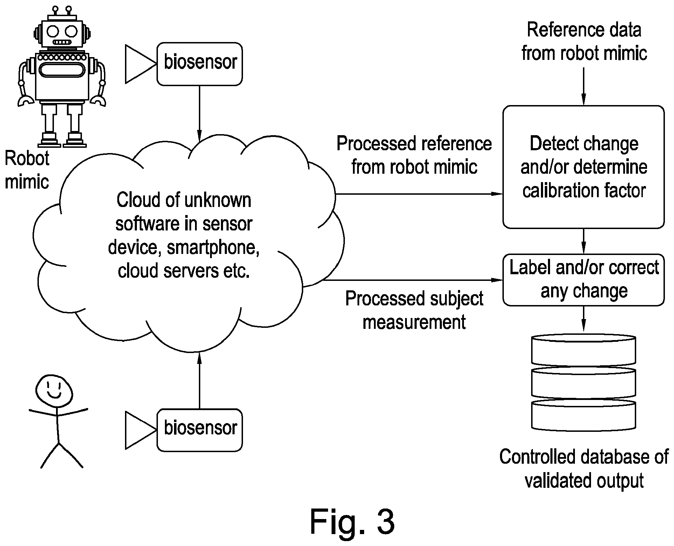

The overall validation of the biosensor measurements requires that measurements from the robot mimic are collected periodically or continuously while the clinical trial subject/patient data is being collected. During this validation process, the same type of biosensor, or biosensor(s), are connected to subject and robot mimic, and the same software cloud is used for the analysis of all data collected. Biosensors used in embodiments of the invention can be any type of biosensor, including consumer grade biosensors "out of the box" as illustrated in figure 3.

Performance of the system can be improved by using multiple robot mimics rather than just one in parallel with the data collection from the subjects.

It is also possible for multiple sensors (eg: different brands of activity watch) to be attached simultaneously to the same robotic mimic to enable the clinical trial subjects / patients to use different sensors in the same study, with standardisation across those sensors provided by the system.

Biosensors that could be validated by embodiments of the invention include, but are not limited to. temperature, blood pressure, speech, activity, and social connectivity (proximity to another sensor.

The invention can also optionally acquire baseline reference data from the subjects or clinical trial subjects under investigation, for example during a set-up phase in a clinical environment while the subject or clinical trial subjects is being observed by a medical professional or a video recording system peforming activities relevant to that sensor.

An embodiment of the invention involves a sensor that measures sleep from patients with a particular pathology (eg: Parkinson's disease), using three axis accelerometers included in a wrist-worn device. This involves the following steps:
1. Collecting representative sensor data from subjects who are undergoing polysomnography (PSG) while wearing sensor A. This data can be used to train a algorithm such that sleep measures derived from sensor A can be made comparable to measures of sleep from PSG.
2. Programming the robotic mimic (a robot arm) with sensor data from the step 1, so that the robot mimic can replicate arm movements during sleep that were equivalent to those movements conducted by the subjects undergoing PSG.

Step 3. Calibration of a different 3-axes accelerometer based biosensor (eg: one that has a longer battery life), sensor B, using the robot mimic programmed in step 2. This calibration would enable sleep measures derived from sensor B to be made comparable to measures of sleep from PSG, just had been done for Sensor A. And this would have been achieved without Sensor B ever having been worn by subjects undergoing PSG.

A second embodiment of the invention involves a biosensor that measures skin temperature and activity and which sends this information via low energy blue-tooth to a smart phone, from where it is sent via either the mobile phone air interface or WiFi to a cloud server, and then via an application programming interface (API) provided by the sensor supplier to a controlled database to be used for research or clinical purposes. During these various data transfers the biosensor data is compressed so that all that is delivered to the controlled database is information on number of steps, amount of deep and light sleep, and skin temperature. It is the data in the controlled database that needs to be validated for the purpose for which it is being used. This involves four stages:
Step 1 - Collecting a representative test sensor dataset: biosensors which collect raw data (eg: actual accelerometer readings rather than steps) are attached to one or several individuals who then undertook the expected range of activities that would be undertaken by the sensor user (walking, running, travelling on public transport and in a car, sleeping, resting etc). The range of movements expected in the population to be studied would be included in this test sensor data, for example different gaits, width of stride etc. Raw data from the sensors will be collected over a sufficient period of time (approx. forty eight hours per subject) to be representative of a subject's behaviour and activities.
Step 2 - Programming a robotic mimic as a sensor phantom: the representative sensor data from step 1 (above) is used to programme the controller of a robot with the appropriate number of degrees of freedom, precise optically encoded motors and with a precisely controlled variable temperature patch. The robotic mimic is programmed with one or more simulated bio-cycles derived from the data collected in step 1. The consumer biosensor(s) are then attached to this robot mimic, so that it collects data that mimics data from sensors attached to an actual clinical trial subject/patient such that the raw data coming out of the biosensor would be highly correlated with the raw data collected in step 1. This robotic mimic is sufficiently precise and reproducible that it can reproduce the same pattern of movement and temperature change each time the simulated bio-cycle is repeated, with errors that are smaller than the acceptable measurement error required for the application. This robotic mimic can also optionally be used to provide absolute calibration of the biosensor measurement.
Step 3 - Collect baseline performance data from the sensor: the biosensor(s) are attached to the robot mimic, operating in a normal mode in which data passes through the software cloud (i.e.: data collected from the biosensor(s) are sent by low energy to a smart phone which sends by WiFi to the cloud server from which it is extracted to the controlled database using an API) while the robot mimic performs the simulated bio-cycle. Baseline data is collected from the output of the software cloud for the sensors, along with test, re-test and inter-device data by repeating the simulated bio-cycle multiple times with multiple versions of the device.
Step 4 - Real-time quality assurance and validation: while biosensor data is being collected from subjects involved in a research study or from subjects in clinical practice, biosensor data from a simulated bio-cycle is also periodically collected (e.g.: daily) from the robot mimic, with the data from both the subjects and robot mimic passing through the same software cloud. The change in measurements from each bio-cycle of the robot mimic is compared to the baseline performance data to detect any change in measurements that is more than a pre-determined difference (eg: 1.5 standard deviations) away from the measured test, re-test performance. If there is any upgrade in the software cloud (e.g..: the software running on the device, or on the smart-phone app, or on the cloud servers) which impacts the measurements, this would be detected from that bio-cycle comparison. The output of the comparison is linked to the subject biosensor data, and can either be used to quantify the magnitude of any change in the sensor output from the biocycle for use in statistical analysis, or can be used to apply a calibration factor to the subject data so that changes in output due to variability in the software cloud are corrected for.

In a further embodiment of the invention, the method allows for authentication of the subject or research subject being studied. According to this embodiment, representative test sensor data is collected from each individual subject or research subject being investigated under standardized conditions (eg: in a clinic) - "subject reference data". This subject reference data may replace or augment the test data described in step 1 above. The system can then compare data collected regulatory or continuously from this subject over time, and compare to the subject reference data. If changes over time are large that indicates that sensor may no longer be monitoring the intended subject and therefore, and if the ongoing measurements are sufficiently consistent with the original subject reference data, that indicates that the same subject is being studied throughout the period. The sensor data might, for example, be the subject's speech, or gait information derived form an actigraphy sensor.

## Claims

1. A method of calibrating sensing data between different sensors of the same type, the method comprising:
a) obtaining physiological data from a test subject through a first sensor;
b) using said physiological data to program a device to replicate physiological attributes exhibited by the test subject;
c) applying a second sensor to the device, the second sensor comprising following upgrades of software or hardware from the first sensor;
d) using the device to replicate the test subject's physiological attributes;
e) obtaining physiological data from the second sensor regularly over time;
f) identifying any variation between the physiological data obtained by the first sensor and the physiological data obtained by the multiple measurements made over time with the second sensor;
g) applying a correction factor to the physiological data obtained from the second sensor in the event that any variation between physiological data obtained from the first sensor and physiological data obtained from the second sensor exceeds a predetermined threshold; and
h) using said correction factor to calibrate the second sensor.

2. A method according to claim 1, wherein the step of using the device to replicate the subject's physiological attributes is repeated multiple times to improve accuracy of replication through machine learning.

3. A method of simultaneously calibrating sensing data from multiple sensors of the same type, the method comprising:
a) obtaining physiological data from a test subject through a first sensor;
b) using said physiological data to program a device to replicate physiological attributes exhibited by the test subject;
c) applying multiple additional sensors to the device, the multiple additional sensors comprising following upgrades of software or hardware from the first sensor;
d) using the device to replicate the test subject's physiological attributes;
e) obtaining physiological data from the additional sensors;
f) identifying any variation between the physiological data obtained by the first sensor and the physiological data obtained by the additional sensors;
g) applying a correction factor to the physiological data obtained from the additional sensors in the event that any variation between physiological data obtained from the first sensor and physiological data obtained from the additional sensors exceeds a predetermined threshold; and
h) using said correction factor to calibrate the additional sensors.

## Patentansprüche

1. Verfahren zum Kalibrieren von Sensordaten zwischen verschiedenen Sensoren des gleichen Typs, wobei das Verfahren Folgendes umfasst:
a) Erlangen physiologischer Daten von einem Testsubjekt durch einen ersten Sensor;
b) Verwenden der physiologischen Daten zum Programmieren einer Vorrichtung, um die physiologischen Eigenschaften, die durch das Testsubjekt gezeigt werden, zu reproduzieren;
c) Anwenden eines zweiten Sensors an der Vorrichtung, wobei der zweite Sensor Verfolgen von Software- oder Hardware-Upgrades des ersten Sensors umfasst;
d) Verwenden der Vorrichtung zum Reproduzieren der physiologischen Eigenschaften des Testsubj ekts;
e) regelmäßiges Erlangen physiologischer Daten von dem zweiten Sensor im Zeitverlauf;
f) Identifizieren jeglicher Abweichungen zwischen den durch den ersten Sensor erlangten physiologischen Daten und den physiologischen Daten, die durch die im Zeitverlauf mit dem zweiten Sensor durchgeführten mehreren Messungen erlangt wurden;
g) Anwenden eines Korrekturfaktors auf die von dem zweiten Sensor erlangten physiologischen Daten für den Fall, dass eine Abweichung zwischen den von dem ersten Sensor erlangten physiologischen Daten und den von dem zweiten Sensor erlangten physiologischen Daten einen vorbestimmten Schwellenwert überschreitet; und
h) Verwenden des Korrekturfaktors zum Kalibrieren des zweiten Sensors.

2. Verfahren nach Anspruch 1, wobei der Schritt des Verwendens der Vorrichtung zum Reproduzieren der physiologischen Eigenschaften des Subjekts mehrere Male wiederholt wird, um die Genauigkeit der Reproduktion durch maschinelles Lernen zu verbessern.

3. Verfahren zum gleichzeitigen Kalibrieren von Sensordaten von mehreren Sensoren des gleichen Typs, wobei das Verfahren Folgendes umfasst:
a) Erlangen physiologischer Daten von einem Testsubjekt durch einen ersten Sensor;
b) Verwenden der physiologischen Daten zum Programmieren einer Vorrichtung, um die physiologischen Eigenschaften, die durch das Testsubjekt gezeigt werden, zu reproduzieren;
c) Anwenden mehrerer zusätzlicher Sensoren an der Vorrichtung, wobei die mehreren zusätzlichen Sensoren Verfolgen von Software- oder Hardware-Upgrades des ersten Sensors umfassen;
d) Verwenden der Vorrichtung zum Reproduzieren der physiologischen Eigenschaften des Testsubj ekts;
e) Erlangen physiologischer Daten von den zusätzlichen Sensoren;
f) Identifizieren jeglicher Abweichungen zwischen den durch den ersten Sensor erlangten physiologischen Daten und den durch die zusätzlichen Sensoren erlangten physiologischen Daten,
g) Anwenden eines Korrekturfaktors auf die von den zusätzlichen Sensoren erlangten physiologischen Daten für den Fall, dass eine Abweichung zwischen den von dem ersten Sensor erlangten physiologischen Daten und den von den zusätzlichen Sensoren erlangten physiologischen Daten einen vorbestimmten Schwellenwert überschreitet; und
h) Verwenden des Korrekturfaktors zum Kalibrieren der zusätzlichen Sensoren.

## Revendications

1. Procédé d'étalonnage de données de détection entre différents capteurs du même type, le procédé comprenant :
a) l'obtention de données physiologiques provenant d'un sujet testé par l'intermédiaire d'un premier capteur ;
b) l'utilisation desdites données physiologiques pour programmer un dispositif afin de reproduire les attributs physiologiques présentés par le sujet testé ;
c) l'application d'un second capteur au dispositif, le second capteur comprenant les mises à niveau suivantes du logiciel ou du matériel provenant du premier capteur ;
d) l'utilisation du dispositif pour reproduire les attributs physiologiques du sujet testé ;
e) l'obtention régulière de données physiologiques en provenance du second capteur au fil du temps ;
f) l'identification de toute variation entre les données physiologiques obtenues par le premier capteur et les données physiologiques obtenues par les multiples mesures effectuées au fil du temps avec le second capteur ;
g) l'application d'un facteur de correction aux données physiologiques obtenues à partir du second capteur dans le cas où toute variation entre les données physiologiques obtenues à partir du premier capteur et les données physiologiques obtenues à partir du second capteur dépasse un seuil prédéfini ; et
h) l'utilisation dudit facteur de correction pour étalonner le second capteur.

2. Procédé selon la revendication 1, ladite étape d'utilisation du dispositif pour reproduire les attributs physiologiques du sujet étant répétée plusieurs fois pour améliorer la précision de la réplication grâce à l'apprentissage automatique.

3. Procédé d'étalonnage simultané de données de détection provenant de multiples capteurs du même type, le procédé comprenant :
a) l'obtention de données physiologiques provenant d'un sujet testé par l'intermédiaire d'un premier capteur ;
b) l'utilisation desdites données physiologiques pour programmer un dispositif afin de reproduire les attributs physiologiques présentés par le sujet testé ;
c) l'application de multiples capteurs supplémentaires au dispositif, les multiples capteurs supplémentaires comprenant les mises à niveau suivantes du logiciel ou du matériel provenant du premier capteur ;
d) l'utilisation du dispositif pour reproduire les attributs physiologiques du sujet testé ;
e) l'obtention de données physiologiques en provenance des capteurs supplémentaires ;
f) l'identification de toute variation entre les données physiologiques obtenues par le premier capteur et les données physiologiques obtenues par les capteurs supplémentaires ;
g) l'application d'un facteur de correction aux données physiologiques obtenues à partir des capteurs supplémentaires dans le cas où toute variation entre les données physiologiques obtenues à partir du premier capteur et les données physiologiques obtenues à partir des capteurs supplémentaires dépasse un seuil prédéfini ; et
h) l'utilisation dudit facteur de correction pour étalonner les capteurs supplémentaires.
